# EUROPEAN PATENT SPECIFICATION

(11) **EP 1 909 855 B1**
(45) Date of publication and mention of the grant of the patent: **07.03.2018**
(21) Application number: 06766165.2
(22) Date of filing: 23.07.2006
(51) Int. Cl.: A61K 51/12, A61N 5/10

(54) **A RADIOACTIVE SURFACE SOURCE AND A METHOD FOR PRODUCING THE SAME**
RADIOAKTIVE OBERFLÄCHENQUELLE UND VERFAHREN ZU IHRER HERSTELLUNG
SOURCE A SURFACE RADIOACTIVE ET PROCEDE DE PRODUCTION CORRESPONDANT

(30) Priority: 26.07.2005 US 702288 P
(43) Date of publication of application: 16.04.2008
(60) Divisional application: 12165220.0
(73) Proprietor: Aplha TAU Medical Ltd., 6997801 Tel-Aviv (IL)
(72) Inventor: KELSON, Itzhak, 63404 Tel Aviv (IL); SCHMIDT, Michael, 53242 Givataim (IL); KEISARI, Yona, Ramat Gan 52364 (IL)
(74) Representative: Dennemeyer & Associates S.A.
(86) International application number: PCT/IL2006/000850
(87) International publication number: WO 2007/013060

(56) References cited:
- EP-A- 1 232 770
- WO-A2-2004/096293
- US-A- 5 342 283
- US-A- 6 030 333
- THOMSON E S ET AL: "STEREOTACTIC MULTIPLE ARC RADIOTHERAPY" BRITISH JOURNAL OF RADIOLOGY, BRITISH INSTITUTE OF RADIOLOGY, LONDON, GB, vol. 63, no. 754, 1990, pages 745-751, XP009033829 ISSN: 0007-1285

## Description

### FIELD AND BACKGROUND OF THE INVENTION

The present invention relates to radiotherapy and, more particularly, to a radioactive surface source capable of emitting decay chain nuclei of a radionuclide.

Cancer is a major cause of death in the modem world. Effective treatment of cancer is most readily accomplished following early detection of malignant tumors. Most techniques used to treat cancer (other than chemotherapy) are directed against a defined tumor site in an organ, such as brain, breast, ovary, colon and the like.

When a mass of abnormal cells is consolidated and is sufficiently large, surgical removal, destruction of the tumor mass using heating, cooling, irradiative or chemical ablation becomes possible because the target is readily identifiable and localizable. Of particular relevance is radiation therapy, also referred to as radiotherapy, or therapeutic radiology, which is used for treating cancer as well as other diseases of the body. Radiotherapy is particularly suitable for treating solid tumors, which have a well-defined spatial contour. Such tumors are encountered in breast, kidney and prostate cancer, as well as in secondary growths in the brain, lungs and liver.

Most radiation treatments are delivered with teletherapy, in which the source of radiation is distant from the target. Such type of treatments typically make use of ionizing radiation, deep tissue-penetrating rays, which can physically and chemically react with diseased cells to destroy them. Each therapy program has a radiation dosage defined by the type and amount of radiation for each treatment session, frequency of treatment session and total number of sessions.

Brachytherapy is a form of radiation therapy in which radioactive pellets or seeds are implanted into or near the target tissue to be treated. The most notable example is the case of prostate cancer, where the entire organ is actually irradiated. Complication rates with brachytherapy are minimal, and are more likely to occur in patients who have undergone transurethral resection of the prostate. Otherwise, patients who undergo transperineal implantation show excellent quality of life. Because the radioactive sources used in brachytherapy deposit all their absorbed dose within a few millimeters of the source, the sources can be arranged so the radiation dose delivered to adjacent normal tissues is minimized, and the dose delivered to the cancerous tissue itself is maximized.

Most commonly, radiotherapy is used as an adjunct way of use, such as treating those remnant, not entirely removed, tumor cells by being exposed to a radiation dose of an external source after the surgical opening of the human body, removal of malignant tumors and the suture of the body parts or radiating the radiation dose directly to the remnant tumor cells before the suture of the body parts involved.

It is well known that different types of radiation differ widely in their cell killing efficiency. Gamma and beta rays have a relatively low efficiency. By contrast, alpha particles as well as other heavy charged particles are capable of transferring larger amount of energy, hence being extremely efficient. In certain conditions, the energy transferred by a single heavy particle is sufficient to destroy a cell. Moreover, the non-specific irradiation of normal tissue around the target cell is greatly reduced or absent because heavy particles can deliver the radiation over the distance of a few cells diameters.

On the other hand, the fact that their range in human tissue is less than 0.1 millimeter, limits the number of procedures in which heavy particles can be used. More specifically, conventional radiotherapy by alpha particles is typically performed externally when the tumor is on the surface of the skin.

International Patent Application, Publication No. WO 2004/096293 to Kelson et al., discloses a radiotherapy method in which a radionuclide, such as, Radium-223, Radium-224, Radon-219 or Radon-220, is positioned in proximity to and/or within the tumor for a predetermined time period. The radionuclide administers a therapeutic dose of decay chain nuclei as well as alpha particles into the tumor. The radionuclide is positioned in proximity to and/or within the tumor either by administering a solution of the radionuclide in a solute to the subject, or by a radiotherapy device, whereby the radionuclide (typically Radium-223 or Radium-224) is on or beneath a surface of the device.

The use of radiotherapy device is for the purpose of confining the radionuclide to the device for its entire lifetime while preventing its convection away from the tumor. However, Radium (both Radium-223 and Radium-224) is known to have high reactivity in water. When the device is brought into contact with the tissue the radioactive atoms interact with body fluids and may be prematurely removed from the device, resulting in decrement of radiation dose delivered to the tumor and, consequently, increment of the radiation dose delivered to undesirable locations.

Furthermore, for such a device to operate efficiently, the radioactive atoms must be close enough to the outer surface of the device to allow their decay products to recoil out of the device with sufficiently high probability to deliver the required alpha dose to the tumor.

The present invention provides solutions to the problems associated with prior art radiotherapy techniques, by providing a radioactive surface source and a method for producing the same.

### SUMMARY OF THE INVENTION

It is against the background, and the limitations and problems associated therewith, that the present invention has been developed.

To achieve this, the method of preparing a radioactive surface source for radiotherapy of the invention comprises the features claimed in claim 1, and the invention provides a radioactive surface source for radiotherapy according to claim 8.

Advantageous embodiments of the invention are claimed in the dependent claims.

According to one aspect of the present invention there is provided a method of preparing a radioactive surface source for radiotherapy. The method comprises: (a) providing a structure having a surface; (b) positioning the structure in a flux of at least one radionuclide so as to collect atoms of the at least one radionuclide on or beneath the surface; and (c) treating the surface such that the atoms are intercalated into the surface but allowed to recoil out of the surface upon radioactive decay.

According to further features in preferred embodiments of the invention described below, the surface is treated by applying thermal treatment thereto. According to still further features in the described preferred embodiments the method further comprises treating the surface with fluid so as to remove residual atoms of the at least one radionuclide from the surface.

According to still further features in the described preferred embodiments the thermal treatment comprises heating the surface to a predetermined temperature selected sufficient to cause diffusion of the atoms below the surface.

According to still further features in the described preferred embodiments the method further comprises, prior to the step (b), coating the surface by at least one layer of polymeric material, such that the atoms are collected into the at least one layer. In this embodiment, the surface is heated to a predetermined temperature selected sufficient to melt the polymeric material thereby to intercalate the atoms into the polymeric material.

According to still further features in the described preferred embodiments the coating the surface is effected by a procedure selected from the group consisting of dipping, spinning, film blowing and injection molding.

According to still further features in the described preferred embodiments the method further comprises, treating the layer(s) of polymeric material with fluid so as to remove residual atoms of the radionuclide(s) from the layer.

According to another aspect of the present invention there is provided a method of preparing a radioactive surface source for radiotherapy. The method comprises: (a) providing a structure made of non-conductive material; (b) at least partially coating the structure by at least one metallic layer thereby forming a metallic surface; and (c) positioning the structure in a flux of at least one radionuclide so as to collect atoms of the at least one radionuclide on or beneath the surface.

According to further features in preferred embodiments of the invention described below, the non-conductive material comprises a bioabsorbable material.

According to further features in preferred embodiments of the invention described below, the method further comprises treating the metallic surface with fluid so as to remove residual atoms of the at least one radionuclide from the surface.

According to still further features in the described preferred embodiments the method further comprises at least partially coating the metallic surface by a protective coat.

According to still further features in the described preferred embodiments the method further comprises treating the protective coat with fluid so as to remove residual atoms of the at least one radionuclide from the protective coat.

According to further features in preferred embodiments of the invention described below, the treatment with fluid is repeated until an amount of the residual atoms is below a predetermined threshold, to ensure that when the surface, protective coat or layer(s) of polymeric material contact the fluid, removal of the atoms from the surface is substantially prevented.

According to still further features in the described preferred embodiments the atoms of the at least one radionuclide are collected by connecting the surface to a voltage source of negative polarity.

According to still further features in the described preferred embodiments the atoms of the at least one radionuclide are collected by direct implantation in a vacuum.

According to still further features in the described preferred embodiments the positioning of the structure in the flux of the at least one radionuclide is performed in a gaseous environment. According to still further features in the described preferred embodiments a pressure of the gaseous environment and a voltage of the voltage source are selected such that the velocity of the atoms is reduced to a thermal velocity.

According to another aspect of the present invention there is provided a radioactive surface source for radiotherapy. The surface source comprises a structure having a surface and atoms of at least one radionuclide being intercalated into the surface but allowed to recoil out of the surface upon radioactive decay. The surface source has the advantage that when it contacts a fluid, e.g., water or blood, removal of the atoms from the surface is substantially prevented.

According to further features in preferred embodiments of the invention described below, the radioactive surface source further comprises at least one layer of polymeric material coating the surface, wherein the atoms of at least one radionuclide are intercalated into the polymeric material.

According to yet another aspect of the present invention there is provided a composition of matter comprises a polymeric material and atoms of at least one radionuclide, wherein the atoms are intercalated into the polymeric material but allowed to recoil out of the polymeric material upon radioactive decay.

According to still another aspect of the present invention there is provided a radioactive surface source for radiotherapy. The radioactive surface source comprises a structure made of a bioabsorbable material, at least partially coated by one or more metallic layers having a surface. The metallic layer comprises atoms of the radionuclide(s) intercalated into the surface but allowed to recoil out of the surface upon radioactive decay.

According to further features in preferred embodiments of the invention described below, the surface source further comprises a protective coat, at least partially coating the metallic layer.

According to still further features in the described preferred embodiments the atoms of the radionuclide(s) are a few angstroms below the surface.

According to still further features in the described preferred embodiments the radionuclide comprises Radium. According to still further features in the described preferred embodiments the Radium is selected from the group consisting of Radium-223 and Radium-224.

According to still further features in the described preferred embodiments the polymeric material comprises a thermoplastic polymeric material.

According to still further features in the described preferred embodiments the polymeric material comprises polymethylmethacrylate.

According to still further features in the described preferred embodiments the radioactive surface source is characterized by radiation dose equivalent of from about 10 to about 100 gray (Gy) in the treated tissue.

According to still further features in the described preferred embodiments the radioactivity of the radionuclide(s) is from about 10 nanoCurie to about 10 microCurie.

According to still further features in the described preferred embodiments the atoms are allowed to emit decay chain nuclei out of the radioactive surface source at an outgoing flux of from about 10² to about 10⁵ nuclei per second.

According to still further features in the described preferred embodiments the surface density of the radionuclide(s) is from about 10¹⁰ to about 10¹³ atoms/cm2.

According to still further features in the described preferred embodiments the structure is made of metal.

According to still further features in the described preferred embodiments the structure is selected from the group consisting of a needle, a wire, a bead, a tip of an endoscope, a tip of a laparoscope and a tip of an imaging device.

The present invention successfully addresses the shortcomings of the presently known configurations by providing a composition of matter, surface source and a method for preparing the same.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWING

The invention is herein described, by way of example only, with reference to the accompanying drawing. With specific reference now to the drawing in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show details of the invention in more detail than is necessary for a fundamental understanding of the invention, the description taken with the drawings making apparent to those skilled in the art how the several forms of the invention may be embodied in practice.

In the drawing:
FIGs. 1-2 are flowchart diagrams of a method for preparing a radioactive surface source for radiotherapy, according to various exemplary embodiments of the present invention;
FIGs. 3a-b are schematic illustrations of a radioactive surface source, in various exemplary embodiments of the invention; and
FIG. 4 is a schematic illustration of a radioactive surface source prepared from a bioabsorbable thread according to the teachings of various exemplary embodiments of the invention.

### DESCRIPTION OF THE PREFERRED EMBODIMENTS

The present embodiments comprise a method, radioactive surface source and composition of matter which can be used in radiotherapy. Specifically, the present invention can be used to locally destroy tumors in either invasive or non-invasive procedures utilizing decay chain nuclei of a radionuclide, such as, but not limited to, Radium-223, Radium-224, Radon-219 and Radon-220.

The principles and operation of a method and device for radiotherapy according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Radiation is a flow of subatomic or atomic particles or waves, which can be emitted by nuclei of a radioactive substance when the nuclei undergo decay processes. One typically encounters four types of radiation: (i) alpha radiation, in a form of helium nuclei, also referred to as alpha particles; (ii) beta radiation, in a form of electrons or positrons, (iii) gamma radiation, in a form of electromagnetic waves or photons; and (iv) neutron radiation, in a form of neutral nucleons.

The rate at which nuclei of a radioactive substance undergo decay and emit radiation is directly proportional to the number of radioactive nuclei in the substance that can decay. Hence, as time goes on, the number of radioactive nuclei in the substance is reduced, and the decay rate decreases. The period of time over which the number of radioactive nuclei of a radioactive substance decreases by a factor of one-half, is referred to as the half-life of the substance. In general, radioactive decay is a quantum mechanical process governed by wavefunctions the square of which is interpreted as probability. In a short period of time, each radioactive nucleus has a certain probability of decaying, but whether it actually does is determined by random chance. When a radioactive nucleus has more than one decay channels, the probability of decaying in a certain channel is referred to as the branching ratio of the channel.

Nuclei which emit alpha particles, also known as alpha emitters, are typically heavy nuclei in which the ratio of neutrons to protons is too low. Following emission of an alpha particle (two protons and two neutrons) from such a nucleus, the ratio is increased and the nucleus becomes more stable. Since the number of protons in the nucleus of an atom determines the element, the loss of an alpha particle actually changes the atom to a different element. For example, Polonium-210 (Po) has 126 neutrons and 84 protons, corresponding to a ratio of 3:2. When an atom of Po-210 emits an alpha particle, the ratio is increased by about 1 %, resulting in a stable Lead-206 (Pb) atom, having 124 neutrons and 82 protons.

Of the aforementioned four types of radiations, alpha particles are the heaviest, about 7000 times the electron's mass, and have the shortest range in human tissue, less than 0.1 millimeter. Conventional radiotherapy procedures by alpha particles are therefore effective only for thin tumors which are on or close beneath the surface of the skin.

*Kelson et al. supra* teach a radiotherapy device having a radioactive surface source in which Radium-223 or Radium-224 atoms are captured on or beneath the surface. The Radium atoms emit alpha particles as well as decay chain nuclei and atoms at sufficient energy to escape the device and destroy or at least damage the tumor.

The present embodiments successfully provide a composition of matter which can be used in the preparation of a radioactive surface source suitable for radiotherapy. The composition of matter comprises a polymeric material and atoms of one or more radionuclides. The atoms of the radionuclide(s) are intercalated into the polymeric material but allowed to recoil out of the polymeric material upon radioactive decay.

"Intercalated atoms" as used herein, refers to foreign atoms which are incorporated into or between molecules of the host material (polymeric material in the present embodiment), in a manner such that there is a bond energy between the atoms and the molecules. As a result of this intercalated state, the host material becomes radioactive in a sense that when a radioactive decay occurs, the daughter nuclei or atoms escape the host material. The intercalated radionuclide atoms are allowed to recoil out of the host material in the sense that the bond energy between the radionuclide atoms and the molecules of the host material is lower than the natural recoil energy of the daughter nuclei or atoms of the radionuclide. Thus, according to the presently preferred embodiment of the invention an intercalated atom remains in its location within the host material until the bond energy is broken by a radioactive decay of the atom itself and/or nearby atoms.

The polymeric material is preferably a thermoplastic polymeric material. Thermoplastic materials are generally materials that flow when heated sufficiently above their glass transition temperature and become solid when cooled. They may be elastomeric or nonelastomeric. Thermoplastic materials useful in the present embodiments include, without limitation, polymethylmethacrylate (PMMA), polyolefins (*e.g*., isotactic polypropylene, polyethylene, polybutylene, polyolefin copolymers or terpolymers such as ethylene/propylene copolymer and blends thereof), ethylene-vinyl acetate copolymers, ethylene acrylic acid copolymers, ethylene methacrylic acid copolymers, polystyrene, ethylene vinyl alcohol, polyesters including amorphous polyester, polyamides, fluorinated thermoplastics such as polyvinylidene fluoride and fluorinated ethylene/propylene copolymers, halogenated thermoplastics such as chlorinated polyethylene and polyether-block-amides.

The composition of the present embodiments can be manufactured by positioning the polymeric material in a flux of the radionuclide so as to collect atoms of the radionuclide on the surface of the polymeric material. Subsequently, the polymeric material can be treated, for example, by applying a thermal treatment, such that the atoms are intercalated into the surface but allowed to recoil out of the surface upon radioactive decay. When a thermal treatment is applied, the thermal treatment preferably comprises heating of the surface so as to melt the polymeric material. The heating can be to a temperature which is sufficiently above the glass transition of the material. For example, when the polymeric material is a (PMMA), it can be heated to a temperature of about 180 °C or more, for a period of about one hour.

As used herein the term "about" refers to ± 10 %.

During the treatment, the atoms of the radionuclide diffuse into the polymeric material. Yet, due to the relatively short mean free path of the radionuclide atoms in the polymeric material (several nanometers for Radium atoms in PMMA), the radionuclide atoms remain relatively close to the surface. When a thermal treatment is employed, the polymeric material is preferably allowed to cool such that the polymeric material becomes solid. The cooling can also be performed using a suitable cooling technique (*e.g*., ventilation, use of artificial cold environment, *etc*.)*.* Once the polymeric material is cooled, the atoms are interlaced in the matrix of the solidified material. Being relatively close to the surface, when the radionuclide atoms decay, the decay products (alpha particle and daughters nuclei or atoms) escape out of the polymeric material.

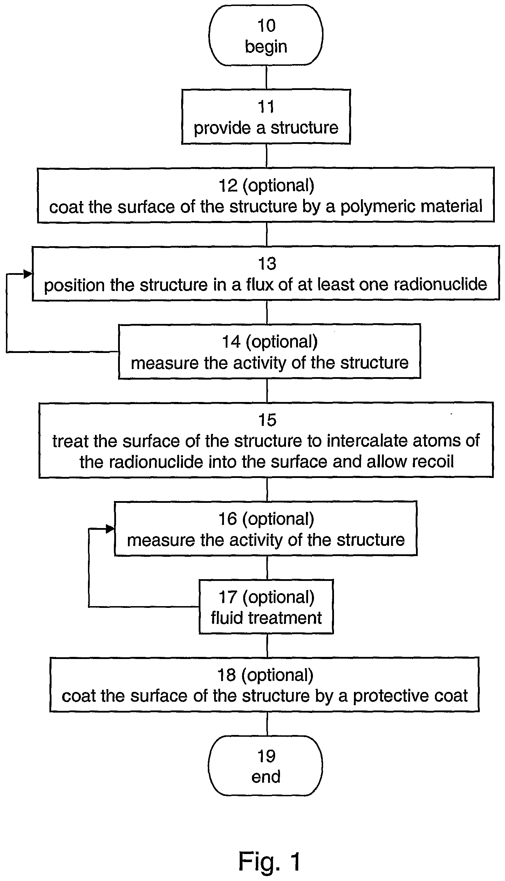

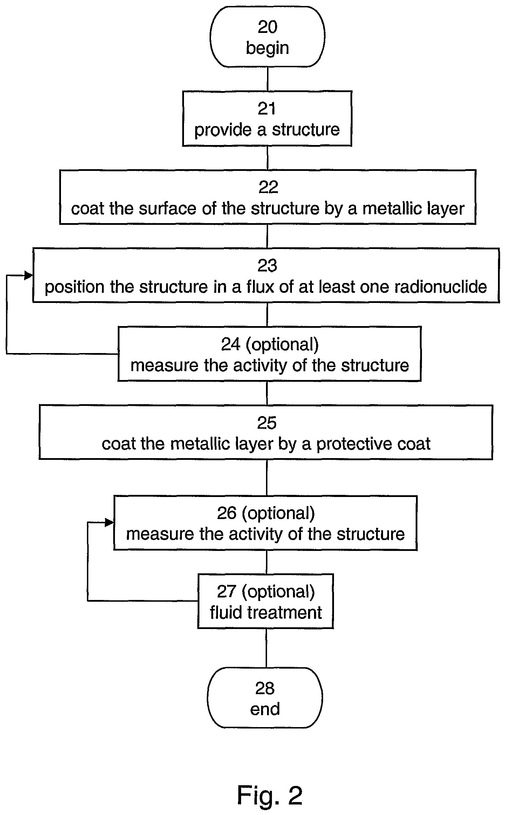

Following is a description of a method which can be used for preparing a radioactive surface source, according to various exemplary embodiments of the present invention. The method is illustrated in the flowcharts of Figures 1 and 2.

It is to be understood that unless otherwise defined the method steps described hereinbelow can be executed either contemporaneously or sequentially in many combinations or orders of execution. Specifically, the ordering of the flowcharts of Figures 1 and 2 is not to be considered as limiting. For example, two or more method steps, appearing in the following description or in the flowcharts in a particular order, can be executed in a different order (*e.g*., a reverse order) or substantially contemporaneously. Additionally, one or more method steps appearing in the following description or in the flowcharts are optional and are presented in the cause of providing what is believed to be a useful and readily understood description of an embodiment of the invention. In this regard, there is no intention to limit the scope of the present invention to the method steps presented in Figures 1 and 2.

Referring to Figure 1, the method begins at step **10** and continues to step **11** in which a structure is provided. The structure can be made of any material. In one embodiment, the structure is made of an electrically conductive material such as, but not limited to, a metal, *e.g.*, stainless steel. In experiments made by the present Inventors, a 316 type stainless steel was used, but other materials can also be used. A preferred procedure for preparing the radioactive surface source using a non conductive structure is provided hereinunder with reference to Figure 2.

The shape of the structure depends on the type of radiotherapy procedure for which the radioactive surface source is prepared. Representative examples include, without limitation, a needle, a wire, a thread (*e.g*., a suture thread), a bead, a tip of an endoscope, a tip of a laparoscope and a tip of an imaging device. The structure is preferably clean in the sense that it is substantially free of residual particles or dust. The residual particles or dust can be removed from the surface of the structure, for example, by cleaning the structure ultrasonically, e.g., in acetone or any other cleaning liquid.

According to a preferred embodiment of the present invention the method continues to optional step **12** in which the surface is coated by one or more layers of polymeric material. The polymeric material enacts the host material into which the atoms of the radionuclide(s) are intercalated to make the host material radioactive. The polymeric material can be any of the above materials. The coating can be done in any way known in the art. In one embodiment of the invention the coating is done by dipping the structure in a solution of the polymeric material and a solvent. For example, it was found by the Inventors of the present invention that a dilute solution of PMMA (*e.g*., several percents, say about 3 %, by weight) in methylisobutylketone (MIBK) is useful for coating the structure.

Other coating techniques suitable for the present embodiments include, without limitation, spinning (*e.g*., electrospinning, wet spinning, gel spinning, dry spinning, melt spinning, dispersion spinning, reaction spinning, tack spinning), film blowing and injection molding.

Whether or not step **12** is performed, the method continues to step **13** in which the structure (with or without the polymeric coat) is positioned in a flux of one or more radionuclides so as to collect atoms thereof on or beneath the surface.

The radionuclide is preferably a relatively short lived radio-isotope, such as, but not limited to, Radium-223 or Radium-224. When Radium 223 is employed, the following decay chain is emitted therefrom:
Ra-223 decays, with a half-life period of 11.4 d, to Rn-219 by alpha emission;
Rn-219 decays, with a half-life period of 4 s, to Po-215 by alpha emission;
Po-215 decays, with a half-life period of 1.8 ms, to Pb-211 by alpha emission;
Pb-211 decays, with a half-life period of 36 m, to Bi-211 by beta emission;
Bi-211 decays, with a half-life period of 2.1 m, to Tl-207 by alpha emission; and
Tl-207 decays, with a half-life period of 4.8 m, to stable Pb-207 by beta emission.

When Radium 224 is employed, the following decay chain is emitted therefrom:
Ra-224 decays, with a half-life period of 3.7 d, to Rn-220 by alpha emission;
Rn-220 decays, with a half-life period of 56 s, to Po-216 by alpha emission;
Po-216 decays, with a half-life period of 0.15 s, to Pb-212 by alpha emission;
Pb-212 decays, with a half-life period of 10.6 h, to Bi-212 by beta emission;
Bi-212 decays, with a half-life of 1h, to Tl-208 by alpha emission (36 % branching ratio), or to Po-212 by beta emission (64 % branching ratio);
Tl-208 decays, with a half-life of 3m, to stable Pb-208 by beta emission; and Po-212 decays, with a half-life of 0.3 µs, to stable Pb-208 by alpha emission.

The collection of the radionuclide on the surface can be achieved through the utilization of a flux generator, such as a flux generating surface source. For example, when the radionuclide is Ra-224, a flux thereof can be generated by a surface source of Th-228. A surface source of Th-228 can be prepared, for example, by collecting Th-228 atoms emitted from a parent surface source of U-232. Such parent surface source can be prepared, for example, by spreading a thin layer of acid containing U-232 on a metal.

Alternatively, a surface source of Th-228 can be obtained by collecting a beam of Fr-228 having a half-life of 39 seconds, which in turn decays to Ra-228. The Ra-228 decays, with a half-life of 5.75 years, to Ac-228 which in turn decays, with a half-life of 6 hours, by beta decay, to Th-228. The entire decay chain, Fr-228, Ra-228, Ac-228 and Th-228 is by beta emission. The population of Th-228 is slowly built over a period of the order of a few years, approaching radioactive equilibrium with Ra-228. Thus, the obtained Th-228 surface source is characterized by the 5.75 years half-life of Ra-228 rather than by its own 1.9 years half-life.

When the radionuclide is Ra-223, a flux thereof can be generated by a surface source of Ac-227, which is in radioactive equilibrium with Th-227. An Ac-227 surface source can be obtained by separating a beam of Fr-227 ions having an energy of a few tens of keV, and implanting the Fr-227 ions in a foil at a depth of a few nanometers. Through a sequence of two short half-life beta decays, the Fr-227 ions decay to Ac-227, thereby providing the desired Ac-227 surface source.

Available isotope separators for separating the Fr-227 or Fr-228 include, without limitation, ISOLDE, located at CERN, Geneva or ISAC, located at TRIUMF, Vancouver.

The collection of the radionuclide on or beneath the surface can be done in more than one way. For example, in one embodiment, the collection is done by electrostatic forces. The desorbing atoms from the flux generator are positively charged (both due to the decay itself and as a result of passage through layers of the flux generator). Thus, by applying a suitable negative voltage between the flux generator and the structure, the desorbing nuclei of the radionuclide can be collected onto the outer surface of the structure. According to a preferred embodiment of the present invention, the collection is done under suitable gas pressure, so as to slow the velocity of the nuclei to a thermal velocity, hence facilitating their collection of the surface of the structure. The electrostatic forces between the structure and the desorbing atoms, results in collection of a considerable amount (e.g., more than 95 %) of the atoms on or beneath the surface of the structure, even when the size of the structure is smaller than the size of the flux generator. Moreover, when the area of the structure is smaller than the area of the flux generator, a high concentration of the radionuclide on the structure can be achieved. Small size structures are advantageous especially in minimal invasive medical procedures. The amount of collected activity depends on the strength of the flux generator, the strength of the electric field between the flux generator and structure and the geometric configuration. Preferably, the surface density of the radionuclide on the surface of the structure is from about 10¹⁰ to about 10¹³ atoms/cm².

In an alternative embodiment, the collection of the radionuclide atoms is by direct implantation in a vacuum. In this embodiment, the flux generator is placed in vacuum in close proximity to the structure. Nuclei or atoms recoiling from the flux generator traverse the vacuum gap and being implanted in the surface of the structure.

In an additional embodiment, the radionuclide can be collected by separating a sufficiently energetic beam of the radionuclide and directing the beam onto the structure or positioning the structure in the path of the beam, so as to allow implanting the radionuclide in the surface of the structure. Radionuclide beams can be obtained, for example, using any of the aforementioned isotope separators.

In various exemplary embodiments of the invention the method continues to step **14** in which the activity of the structure is measured. The measurement can be done using an alpha counting setup and a constant air stream which removes daughters atoms (*e.g*., Radon atoms) desorbing from the surface of the structure. The measurement of the characteristic alpha particles of the radionuclide gives the overall activity of the structure, while the measurement of alpha particles emitted by the daughter atoms remaining in the wire yields the desorption probability of the daughter atoms from the surface. When the structure is not coated by polymeric material, the desorption probabilities of daughter atoms are about 45 to 55 %. When the structure is coated by polymeric material, the desorption probabilities are higher (about 75 to 85 %) because many of the daughter atoms recoiling inward diffuse out through the semi porous, loosely packed polymeric layer.

If the activity of the structure is too low, the method can loop back to step *13* for collecting more atoms. If the activity of the structure is too high, excess radionuclide atoms can be washed by contacting the structure with washing liquid such as water. Alternatively, the radionuclide can be allowed to decay without intervening until the desired activity is achieved.

Once the radionuclide is collected on the surface of the structure, the method continues to step **15** in which the surface of the structure is treated such that atoms of the radionuclide are intercalated into the surface but allowed to recoil out of the surface upon radioactive decay.

In various exemplary embodiments of the invention the treatment comprises a thermal treatment, which generally includes heating followed by cooling. In the embodiments in which the surface is coated by a polymeric material, the heating is done so as to melt the polymeric material. Such heating results in diffusion and intercalation of the radionuclide atoms into the layer(s) of the polymeric material as further detailed above. The subsequent cooling (either spontaneously or via an active cooling technique) results in the interlacement of the radionuclide atoms in the solidified polymeric material.

In the embodiments in which there is no coating of the surface by polymeric material, the heating is preferably to a temperature selected sufficient to cause diffusion of atoms below surface, such that the radionuclide atoms are intercalated into the surface of the structure. Thus, in this embodiment, the host material is enacted by the structure itself. A typical temperature in this embodiment is from about 400 °C to about 500 °C.

Optionally and preferably the method comprises one or more steps designed to minimize non-radioactive removal of radionuclide atoms from the surface of the structure.

Hence, according to a preferred embodiment of the present invention the method continues to optional step **16** in which the activity of the structure is measured as further detailed hereinabove. At this stage, the activity of the radionuclide on the structure is approximately the same, except for characteristic exponential decay with time. The desorption probabilities of daughter atoms are about 45 to 55 % both with and without polymeric coating due to the suppression of daughter atoms diffusion through the structure or solidified coat.

The method can then continue to optional step **17** in which the surface is treated with fluid so as to remove residual atoms of the radionuclide therefrom. The treatment can comprise, for example, the immersion of the structure in warm water (about 40 °C), for a predetermined duration, say, 30 minutes or more.

The method can then loop back to step **16** so as to estimate the amount of residual radionuclide atoms which were removed during the fluid treatment. Typically, the first fluid treatment results in activity reduction of from about 5 % to about 20 %.

According to a preferred embodiment of the present invention the method loops between step **17** and step **16** until the amount of residual atoms is below a predetermined threshold, which can correspond, for example, to an activity reduction of less than 2 % during the fluid treatment. As will be appreciated by one ordinarily skilled in the art, such repetition ensures that when the surface contacts fluid, removal of atoms from surface is substantially prevented.

Optionally and preferably, the method can proceed to step **18** in which the surface of the structure is coated by a protective coat, which may be, for example, a thin (*e.g*., a few nanometers in thickness, say 5 nanometers) layer of Titanium. The protective coat serves for further protecting the surface from shedding the radionuclide atoms in a non-radioactive fashion. The protective coat is preferably selected so as not to prevent emission of alpha particles and other decay chain products from the surface of the structure. In the embodiments in which step **18** is executed, it can be executed irrespectively of step **12** above. Thus, the method according to the present embodiments contemplates execution or omission of any of steps **12** and **18.**

The method ends at step **19.**

Figure 2 is a flowchart diagram of a method suitable for preparing a radioactive surface source according to other exemplary embodiments of the present invention. This method is particularly useful when the structure carrying the radionuclide(s) is non conductive.

The method begins at step **20** and continues to step **21** in which a structure is provided. The structure is preferably non conductive. More preferably, the structure is made of a bioabsorbable material, which can be naturally occurring material, synthesized material or combination of naturally occurring and synthesized material. Representative examples of bioabsorbable materials include, without limitation, collagen and polymers of glycolide, lactide, caprolactrone, p-dioxanone, trimethylene carbonate and physical and chemical combinations thereof.

The advantage of using a radioactive surface source made of a bioabsorbable material is that it can be implanted near or in the target location and allowed to be absorbed in the host tissue without having to remove it after treatment.

The method continues to step **22** in which the structure is at least partially coated by a metallic layer, which is preferably from about 5 nanometers to about 100 nanometers in thickness. The metallic layer serves for providing the structure with sufficient electrical conductivity to facilitate the collection of the radionuclide on or beneath the layer. The metallic layer can be made of any metal or metal alloy, such as a transition metal, a rare earth metal, an alkali metal or an alloy of two or more metals. In a preferred embodiment, the metallic layer is made of titanium. According to a preferred embodiment of the present invention the metallic layer partially coats the structure so as to allow the structure to interact with body fluids upon implantation. This embodiment is particularly useful when the structure is made of bioabsorbable material, whereby the uncoated parts of the bioabsorbable material are degraded and absorbed by the host tissue.

The method continues to step **23** in which the structure is positioned in a flux of one or more radionuclides so as to collect atoms thereof on or beneath the surface, as further detailed hereinabove. According to a preferred embodiment of the present invention the method continues to step **24** in which the activity of the structure is measured. If the activity of the structure is too low, the method can loop back to step **23** for collecting more atoms, conversely, if the activity is too high, excess radionuclide atoms can be washed or allowed to decay, as further detailed hereinabove.

The method continues to step **25** in which the metallic layer is coated by a protective coat, which is typically from about 5 nanometers to about 20 nanometers in thickness. The protective coat can be made of any material suitable for protecting the metallic layer from shedding the radionuclide atoms in a non-radioactive fashion, and, at the same time not prevent emission of alpha particles and other decay chain products from the metallic layer. Representative examples of materials suitable for the protecting coat include, without limitation, a biostable material and metal, e.g., Titanium.

Similarly to the above embodiments, the method, optionally and preferably, comprises one or more steps designed to minimize non-radioactive removal of radionuclide atoms from the surface of the structure. Thus, in various exemplary embodiments of the invention the method continues to steps **26** and **27** in which the activity of the structure is measured (step **26**) and the structure is treated with fluid to remove residual atoms of the radionuclide (step **27**). Steps **26** and **27** can be repeated until the amount of residual atoms is below a predetermined threshold, as further detailed hereinabove.

The method ends at step **28.**

Execution of selected steps of the above method according to present embodiments successfully produces a radioactive surface source for radiotherapy, in which the atoms of the radionuclide(s) are intercalated into the surface and/or the polymeric material matrix (in the embodiments in which such matrix is provided) but allowed to recoil out of the surface upon radioactive decay.

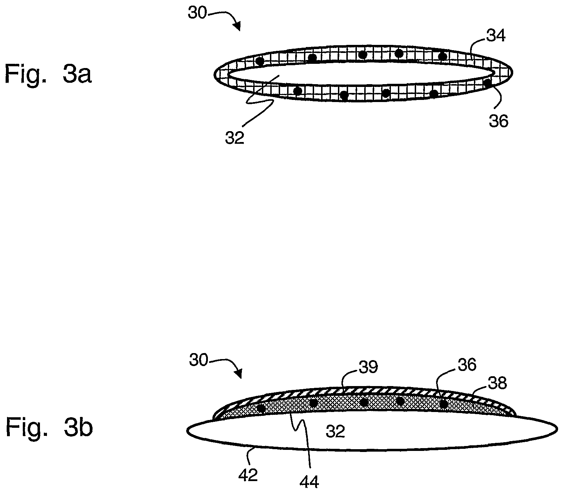

Figures 3a-b are schematic illustrations of a radioactive surface source **30** prepared according to the teachings of the present embodiments. In the exemplified embodiment shown in Figure 3a, source **30** comprises a structure **32** at least partially coated by a polymeric material **34,** and atoms **36** of one or more radionuclides intercalated into polymeric material **34.** In the exemplified embodiment shown in Figure 3b, structure **32** is partially coated by a metallic layer **38** and atoms **36** are intercalated into layer **38.** This embodiment is particularly useful when structure **32** is made of non-conductive material, e.g., bioabsorbable material. As shown, structure **32** includes exposed parts **42** and coated parts **44.** As stated, when structure **32** is made of bioabsorbable material, the exposed parts are degraded and absorbed by the host tissue. Also shown in Figure 3b is a protective coat **39,** at least partially coating layer 38.

In use, the surface source of the present embodiments is brought in proximity or into a tumor, and the radioactive emission is not reduced due to the contact between the surface and the blood or tissue of the subject. Only daughter atoms are released into the surrounding environment and dispersed therein by thermal diffusion and/or by convection via body fluids. The daughter atoms and their massive decay products (*i.e*., alpha particles and other daughters nuclei), either interact with the cells of the tumor or continue the decay chain by producing smaller mass particles. As will be appreciated by one ordinarily skilled in the art, the close proximity between the radionuclide and the tumor, and the large number of particles which are produced in each chain, significantly increase the probability of damaging the cells of interest, hence allowing for an efficient treatment of the tumor.

The surface source of the present embodiments can be utilized either as a stand alone radiotherapy procedure or in combination with conventional debulking procedures for surgically removing or ablating a tumor. In typical conventional debulking procedures, once the tumor is removed, remnants of the tumor may be still present in tissue surrounding the region which was surgically removed or ablated. Hence, according to a preferred embodiment of the present invention the surface source can be positioned in proximity or within the surrounding tissue, again, for a predetermined time period, so as to administer the decay chain nuclei and alpha particles into the surrounding tissue.

The amount of radiation provided by the surface source to the tissue is preferably from about 10 to about 100 gray (Gy) in the treated tissue. In terms of particles flux, the outgoing flux of the decay chain emitted from the surface source of the present embodiments is preferable from about 10² to about 10⁵ atoms/sec, more preferably from about 10³ to about 10⁴ atoms/sec.

The activity of the surface source of the present embodiment is preferably selected so as to allow the administration of a therapeutic dose into the tumor. The relation between the activity of the surface source and the administered dose may depend on many factors such as, but not limited to, the type and size of the tumor, the number of locations to which the surface source is inserted (when more than one surface source is used), the distance between the surface source and the tumor and the like. A preferred activity of the surface source of the present embodiments is, without limitation, from about 10 nanoCurie to about 10 microCurie, more preferably from about 10 nanoCurie to about 1 microCurie.

The surface source of the present embodiments can be used to destroy many tumors and to treat many types of cancer. Representative examples generally include, without limitation, lung, breast and brain cancers. Other examples include, without limitation, neuroblastoma, thyroid gland tumor, gestational trophoblastic tumor, uterine sarcoma, carcinoid tumor, colon carcinoma, esophageal carcinoma, hepatocellular carcinoma, liver carcinoma, lymphoma, plasma cell neoplasm, mesothelioma, thymoma, alveolar soft-part sarcoma, angiosarcoma, epithelioid. sarcoma, extraskeletal chondrosarcoma, fibrosarcoma, leiomyosarcoma, liposarcoma, malignant fibrous histiocytoma, malignant hemangiopericytoma, malignant mesenchymoma, malignant schwannoma, synovial sarcoma, melanoma, neuroepithelioma, osteosarcoma, leiomyosarcoma, Ewing sarcoma, osteosarcoma, rhabdomyo-sarcoma, hemangiocytoma, myxosarcoma, mesothelioma (*e.g*., lung mesothelioma), granulosa cell tumor, thecoma cell tumor and Sertoli-Leydig tumor.

Hence, the surface source of the present embodiments can be used to treat many types of cancers, such as, but not limited to, vaginal cancer, vulvar cancer, cervical cancer, endometrial cancer, ovarian cancer, rectal cancer, salivary gland cancer, laryngeal cancer, nasopharyngeal cancer, many lung metastases and acute or chronic leukemia (*e.g*., lymphocytic, Myeloid, hairy cell).

Additional objects, advantages and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions illustrate the invention in a non limiting fashion.

### Preparation of a Bioabsorbable Radioactive Surface Source

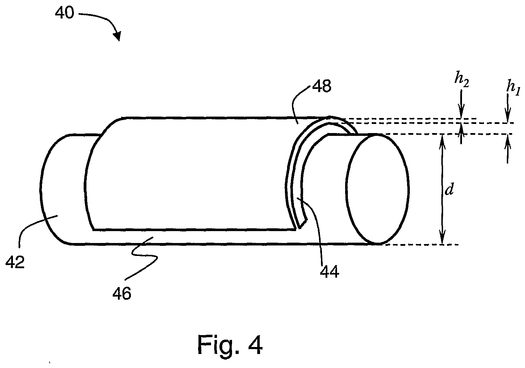

A prototype bioabsorbable radioactive surface source 40 was prepared in accordance with various exemplary embodiments of the invention. The prototype surface source is schematically illustrated in Figure 4. A 3-0 gauge (diameter d of about 0.3 millimeter) bioabsorbable suture thread ***42*** was used as the structure of the surface source. The thread was made of a monofilament glycomer (No. 631; model GM-332 of BIOSYN™).

The thread was coated by Titanium in an RF sputtering system to form a first titanium layer ***44.*** The maximal thickness *h*₁ of layer ***44*** was about 300 angstroms. A sector ***46*** of approximately 90 degrees at the back side of the tread was left uncoated.

A 4 millimeter length of the coated thread was positioned in a flux of Ra-224 ions recoiling from a Th-228 generator. Following a collection period of 46.5 hours, the Ra-224 activity and the desorption probability of Rn-220 from the source were measured. The measurement was carried out in a standard alpha-particle counting chamber, with a constant air-flow removing the desorbing Rn-220 atoms. The desorption probability was determined from the ratio of residual Rn-220 counts to the Ra-224 counts in the source. The total Ra-224 activity was 180 ± 7 nanoCuries and the Rn-220 desorption probability was 48 ±4%. The overall activity represented a collection efficiency of about 80 % of the Ra-224 atoms emitted by the Th-228 generator. The Rn-220 desorption probability corresponded well to the theoretical value of 50 % expected from atoms decaying on the outermost layer of a surface.

A thin layer ***48*** of Titanium was deposited on layer ***44*** by RF sputtering. Layer ***48*** served as the protective coat of surface source ***40.*** During the RF sputtering, the orientation of the thread was selected such as to align layer ***48*** onto layer ***44*** but not on the exposed sector ***46.*** The maximal thickness *h*₂ of layer ***48*** was about 150 angstroms.

Surface source was immersed in water at room temperature to remove loose Titanium particles and residual Ra-224 atoms. Following 40 minutes of immersion, the Ra-224 activity and the desorption probability of Rn-220 from the source were measured in the alpha-particle counting chamber. The Ra-224 activity was reduced by 9 % ± 4 %, and the Rn-220 desorption probability was 19 % ± 4 %. The reduction in the Rn-220 desorption probability is explained by the presence of the metallic protective coat ***48*** which prevents a portion of the Rn-220 atoms to penetrate therethrough.

Subsequently to the activity measurements, the prototype surface source was immersed again in water at room temperature for a period of 19 hours. The activity was re-measured in the alpha counting chamber. Taking into account the trivial change due to the half-life of Ra-224 (3.66 days), the change in the Ra-224 activity was negligible (0 % ± 3 %) and the Rn-220 desorption probability was 26 % ± 4 %. It was therefore demonstrated that when the radioactive surface source of the present embodiments contact water, removal of the atoms from the surface is substantially prevented.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Citation or identification of any reference in this application shall not be construed as an admission that such reference is available as prior art to the present invention.

## Claims

1. A method of preparing a radioactive surface source for radiotherapy, comprising:
(a) providing a structure having a surface;
(b) coating said surface with at least one layer of polymeric material;
(c) positioning said structure in a flux of at least one radionuclide selected from the group consisting of Radium-223 and Radium-224, so as to collect atoms of said at least one radionuclide into said at least one layer; and
(d) heating said polymeric material to a predetermined temperature selected sufficient to melt said polymeric material, such that said atoms are intercalated into said polymeric material but allowed to recoil out of said polymeric material upon radioactive decay.

2. The method of claim 1, further comprising treating said at least one layer of polymeric material with fluid so as to remove residual atoms of said at least one radionuclide from said at least one layer.

3. The method of claim 1, wherein said coating said surface is effected by a procedure selected from the group consisting of dipping, spinning, film blowing and injection molding.

4. The method of claim 1, wherein said structure is made of non-conductive material, and said step (a) further comprises at least partially coating said structure by at least one metallic layer thereby forming said surface.

5. The method of claim 1, wherein said atoms of said at least one radionuclide are collected by direct implantation in a vacuum.

6. The method of claim 1, wherein said atoms of said at least one radionuclide are collected by connecting said surface to a voltage source of negative polarity.

7. The method of claim 6, wherein said positioning of said structure in said flux of said at least one radionuclide is performed in a gaseous environment.

8. A radioactive surface source for radiotherapy, comprising a structure coated by at least one layer of polymeric material and atoms of at least one radionuclide is selected from the group consisting of Radium-223 and Radium-224, the radioactive surface source is obtained by the method according to any of claims 1-7.

9. The method or radioactive surface source of any of claims 1-8, wherein said polymeric material comprises a thermoplastic polymeric material.

10. The method or radioactive surface source of any of claims 1-8, wherein said polymeric material comprises polymethylmethacrylate.

11. The method or radioactive surface source of any of claims 1-10, wherein the radioactive surface source is **characterized by** radiation dose equivalent of from about 10 to about 100 gray in a treated tissue.

12. The method or radioactive surface source of any of claims 1-11, wherein a radioactivity of said at least one radionuclide is from about 10 nanoCurie to about 10 microCurie.

13. The method or radioactive surface source of any of claims 1-12, wherein said atoms are allowed to emit decay chain nuclei out of the radioactive surface source at an outgoing flux of from about 10² to about 10⁵ nuclei per second.

14. The method or radioactive surface source of any of claims 1-13, wherein a surface density of said at least one radionuclide is from about 10¹⁰ to about 10¹³ atoms/cm².

15. The method or radioactive surface source of any of claims 1-13, wherein said structure is made of metal.

16. The method or radioactive surface source of any of claims 1-15, wherein said structure is selected from the group consisting of a needle, a wire, a bead, a tip of an endoscope, a tip of a laparoscope and a tip of an imaging device.

17. The method or radioactive surface source of any of claims 1-15, wherein said structure is a suture thread.

## Patentansprüche

1. Verfahren zum Herstellen einer radioaktiven Oberflächenquelle zur Strahlentherapie, das umfasst:
(a) Bereitstellen einer Struktur mit einer Oberfläche;
(b) Beschichten der Oberfläche mit zumindest einer Schicht aus polymerem Material;
(c) Positionieren der Struktur in einem Fluss von zumindest einem Radionuklid, das aus der Gruppe ausgewählt ist, bestehend aus Radium-223 und Radium-224, um Atome des zumindest einen Radionuklids in die zumindest eine Schicht zu sammeln; und
(d) Erhitzen des polymeren Materials auf eine vordefinierte Temperatur, die so ausgewählt wird, dass sie ausreicht, um das polymere Material zu schmelzen, so dass die Atome in das polymere Material eingefügt werden, bei radioaktivem Zerfall jedoch aus dem polymeren Material austreten dürfen.

2. Verfahren nach Anspruch 1, das ferner das Behandeln der zumindest einen Schicht aus polymerem Material mit Fluid umfasst, um Restatome des zumindest einen Radionuklids aus der zumindest einen Schicht zu entfernen.

3. Verfahren nach Anspruch 1, wobei die Beschichtung der Oberfläche durch eine Verfahrensweise erfolgt, die aus der Gruppe ausgewählt ist, bestehend aus Tauchformen, Spinnformen, Folienblasformen und Spritzguss.

4. Verfahren nach Anspruch 1, wobei die Struktur aus nichtleitfähigem Material hergestellt ist und Schritt (a) ferner das zumindest teilweise Beschichten der Struktur mit zumindest einer metallischen Schicht umfasst, um die Oberfläche zu bilden.

5. Verfahren nach Anspruch 1, wobei die Atome des zumindest einen Radionuklids durch direktes Implantieren in einem Vakuum gesammelt werden.

6. Verfahren nach Anspruch 1, wobei die Atome des zumindest einen Radionuklids durch Verbinden der Oberfläche mit einer Spannungsquelle negativer Polarität gesammelt werden.

7. Verfahren nach Anspruch 6, wobei das Positionieren der Struktur in dem Fluss des zumindest einen Radionuklids in einer gasförmigen Umgebung durchgeführt wird.

8. Radioaktive Oberflächenquelle zur Strahlentherapie, die eine Struktur umfasst, die mit zumindest einer Schicht aus polymerem Material und Atomen zumindest eines Radionuklids beschichtet ist, ausgewählt aus der Gruppe, bestehend aus Radium-223 und Radium 224, wobei die radioaktive Oberflächenquelle mit dem Verfahren nach einem der Ansprüche 1 bis 7 erhalten wird.

9. Verfahren oder radioaktive Oberflächenquelle nach einem der Ansprüche 1 bis 8, wobei das polymere Material ein thermoplastisches polymeres Material umfasst.

10. Verfahren oder radioaktive Oberflächenquelle nach einem der Ansprüche 1 bis 8, wobei das polymere Material Polymethylmethacrylat umfasst.

11. Verfahren oder radioaktive Oberflächenquelle nach einem der Ansprüche 1 bis 10, wobei die radioaktive Oberflächenquelle durch eine Strahlendosis äquivalent zu ungefähr 10 bis ungefähr 100 Gray in einem behandelten Gewebe gekennzeichnet ist.

12. Verfahren oder radioaktive Oberflächenquelle nach einem der Ansprüche 1 bis 11, wobei eine Radioaktivität des zumindest einen Radionuklids ungefähr 10 Nanocurie bis ungefähr 10 Mikrocurie ist.

13. Verfahren oder radioaktive Oberflächenquelle nach einem der Ansprüche 1 bis 12, wobei die Atome Zerfallskettenkerne aus der radioaktiven Oberflächenquelle bei einem ausströmenden Fluss von ungefähr 10² bis ungefähr 10⁵ Kernen pro Sekunde ermittieren dürfen.

14. Verfahren oder radioaktive Oberflächenquelle nach einem der Ansprüche 1 bis 13, wobei eine Oberflächendichte des zumindest einen Radionuklids ungefähr 10¹⁰ bis ungefähr 10¹³ Atome/cm² beträgt.

15. Verfahren oder radioaktive Oberflächenquelle nach einem der Ansprüche 1 bis 13, wobei die Struktur aus Metall hergestellt ist.

16. Verfahren oder radioaktive Oberflächenquelle nach einem der Ansprüche 1 bis 15, wobei die Struktur aus der Gruppe ausgewählt ist, bestehend aus einer Nadel, einem Draht, einer Perle, einer Spitze eines Endoskops, einer Spitze eines Laparoskops und einer Spitze einer B ildgebungsvorrichtung.

17. Verfahren oder radioaktive Oberflächenquelle nach einem der Ansprüche 1 bis 15, wobei die Struktur ein Nahtfaden ist.

## Revendications

1. Procédé de préparation à la radiothérapie d'une source à surface radioactive, comprenant :
(a) la fourniture d'une structure possédant une surface ;
(b) le revêtement de ladite surface avec au moins une couche de matière polymère ;
(b) le positionnement de ladite structure dans un flux d'au moins un nucléide radioactif choisi dans le groupe constitué de Radium 223 et de Radium 224, de sorte à collecter les atomes dudit au moins un nucléide radioactif dans ladite au moins une couche ; et
(d) le chauffage de ladite matière polymère à une température prédéterminée choisie suffisante pour faire fondre ladite matière polymère, de telle sorte que lesdits atomes soient intercalés dans ladite matière polymère mais qu'ils puissent ressortir de ladite matière polymère sur détérioration radioactive.

2. Procédé selon la revendication 1, comprenant en outre le traitement d'au moins une couche de matière polymère avec du fluide de sorte à éliminer les atomes résiduels dudit au moins un radionucléide de ladite au moins une couche.

3. Procédé selon la revendication 1, dans lequel ledit revêtement de ladite surface est effectué par une procédure choisie dans le groupe constitué du trempage, de l'application centrifuge, du soufflage de feuille et du moulage par injection.

4. Procédé selon la revendication 1, dans lequel ladite structure est constituée de matière non conductrice, et ladite étape (a) comprend en outre le revêtement au moins partiel de ladite structure au moyen d'au moins une couche métallique formant ainsi ladite surface.

5. Procédé selon la revendication 1, dans lequel lesdits atomes dudit au moins un radionucléide sont collectés par implantation directe dans un vide.

6. Procédé selon la revendication 1, dans lequel lesdits atomes dudit au moins un radionucléide sont collectés en reliant ladite surface à une source de tension de polarité négative.

7. Procédé selon la revendication 6, dans lequel ledit positionnement de ladite structure dans ledit flux dudit au moins un radionucléide est effectué dans un environnement gazeux.

8. Source à surface radioactive pour radiothérapie, comprenant une structure revêtue d'au moins une couche de matière polymère et des atomes d'au moins un radionucléide choisi dans le groupe constitué du Radium 223 et du radium 224, la source à surface radioactive étant obtenue par le procédé selon l'une quelconque des revendications 1 à 7.

9. Procédé ou source à surface radioactive selon l'une quelconque des revendications 1 à 8, dans lequel/laquelle ladite matière polymère comprend une matière polymère thermoplastique.

10. Procédé ou source à surface radioactive selon l'une quelconque des revendications 1 à 8, dans lequel/laquelle ladite matière polymère comprend du polyméthylméthacrylate.

11. Procédé ou source à surface radioactive selon l'une quelconque des revendications 1 à 10, dans lequel/laquelle la source à surface radioactive est **caractérisée par** un équivalent de dose de rayonnement d'environ 10 à environ 100 gray dans un tissu traité.

12. Procédé ou source à surface radioactive selon l'une quelconque des revendications 1 à 11, dans lequel/laquelle une radioactivité dudit au moins un radionucléide est d'environ 10 nanoCurie à environ 10 microCurie.

13. Procédé ou source à surface radioactive selon l'une quelconque des revendications 1 à 12, dans lequel/laquelle lesdits atomes sont autorisés à émettre des noyaux de chaîne de désintégration à partir de la source à surface radioactive à un flux sortant d'environ 10² à environ 10⁵ noyaux par seconde.

14. Procédé ou source à surface radioactive selon l'une quelconque des revendications 1 à 13, dans lequel/laquelle une densité de surface dudit au moins un radionucléide est d'environ 10¹⁰ à environ 10¹³ atomes/cm².

15. Procédé ou source à surface radioactive selon l'une quelconque des revendications 1 à 13, dans lequel/laquelle ladite structure est constituée de métal.

16. Procédé ou source à surface radioactive selon l'une quelconque des revendications 1 à 15, dans lequel/laquelle ladite structure est choisie dans le groupe constitué d'une aiguille, d'un fil, d'une bille, d'une pointe d'un endoscope, d'une pointe d'un laparoscope et d'une pointe d'un dispositif d'imagerie.

17. Procédé ou source à surface radioactive selon l'une quelconque des revendications 1 à 15, dans lequel ladite structure est un fil de suture.
